# EUROPEAN PATENT APPLICATION

(11) **EP 3 246 004 A1**
(43) Date of publication of application: **22.11.2017**
(21) Application number: 17171451.2
(22) Date of filing: 17.05.2017
(51) Int. Cl.: A61K 8/34, A61K 8/73, A61Q 19/00, A61K 8/97

(54) **METHOD OF PREPARING FUNCTIONAL BIOCELLULOSE MEMBRANES AND THE MASK PREPARED THEREBY**

(30) Priority: 17.05.2016 US 201662337673 P
(71) Applicant: TCI Co., Ltd., Taipei 11494 (TW)
(72) Inventor: LIN, Yung-Hsiang, 11494 Taipei (TW); HO, Cheng-Yu, 11494 Taipei (TW)
(74) Representative: dompatent von Kreisler Selting Werner - Partnerschaft von Patent- und Rechtsanwälten mbB

(57) **Abstract**

The present invention provides a method of preparing a functional biocellulose membrane, including immersing a biocellulose membrane in a functional solution and elevating a temperature of the functional solution to 35°C or higher for a period of time. The present invention also provides a functional biocellulose mask produced according to the method. The functional biocellulose mask is filled uniformly with functional ingredients and achieves outstanding skin care effects.

## Description

### CROSS-REFERENCE TO RELATED APPLICATION

This application claims priority of Provisional Application No. 62/337,673, filed on May 17, 2016, the content of which is incorporated herein in its entirety by reference.

### BACKGROUND OF THE INVENTION

### 1. Field of the Invention

The present invention relates to a method of preparing biocellulose membranes. Particularly, the present invention relates to a method of preparing functional biocellulose membranes and the functional biocellulose mask prepared thereby.

### 2. The Prior Art

As the global economy develops, fulfillment of basic needs in life is not the only concern for modem people, recreation and beauty care issues are also paid seriously attention to. In order to maintain or regain youthful appearance, both women and men are enthusiastic about doing body care and beauty care, including taking care of the body's interior and exterior, especially the face. The most common method of achieving this goal is the use of care products by way of smearing or spray. Therefore, facial care products have become the principle focus of research and development for related manufacturers.

Applying facial mask on face is a general practice to enhance the effectiveness of caring ingredients. The principle of facial masks is to force the pores on face to open through its sealing action, allowing for better absorption of the caring ingredients into the skin in comparison with direct application of products containing those ingredients. Usually, application of a facial mask for 15∼20 minutes provides brightness and refreshment to the facial skin.

For different purposes, facial masks may contain ingredients for whitening, skin tightening, and moisturizing and have a moisture condition. When such facial masks are applied to users' faces, the caring ingredients gradually penetrate the facial skin to achieve the skin care effects.

There are multiple types of facial masks in the current market, including masks in the forms of fibrous membrane, liquid, gel, lotion, cream, and paste. Among these, the most commonly used is fibrous membrane-based facial masks. The fibrous membrane-based facial masks can vary in terms of the effects of the loaded essences and the mask materials. Regarding the effects of the loaded essences, different ingredients, such as wine polyphenols, cucumber extract, aloe extract, milk, yogurt, and fruit extracts, are selected for addition to the facial masks with specific purposes. Regarding the mask materials, biocellulose membranes are suitable for manufacture of facial masks, because biocellulose membranes are soft-touching and possess delicate structure and excellent absorbency and flexibility.

One disadvantage of biocellulose membranes as facial masks is uneven distribution of the essences on the surface of biocellulose membranes, which is resulted from addition of the essences immediately before packaging of the biocellulose membranes. The characteristics of biocellulose membranes cause the essences difficult to spread evenly on the surface, and thus a loss of caring effects on some parts of the face is often experienced by the users.

### SUMMARY OF THE INVENTION

In one aspect, the present invention provides a method of preparing a functional biocellulose membrane, including immersing a biocellulose membrane in a functional solution, and elevating a temperature of the functional solution to 35°C or higher for a period of time.

In one embodiment of the present invention, the functional solution includes at least a polyphenol-based functional ingredient. The polyphenol-based functional ingredient is selected from the group consisting of tea polyphenols such as catechin, anthocyanidins, and isoflavones.

In another embodiment of the present invention, the functional solution further includes at least a pigment selected from the group consisting of lutein, carotenes, and carotenoids such as lycopene.

In yet another embodiment of the present invention, the temperature for the step of immersing is 35°C∼37°C, and the period of time is 30∼180 minutes.

In another aspect, the present invention also provides a functional biocellulose mask, which is a biocellulose membrane prepared according to the method previously described and has a facial shape, and it includes a plurality of openings corresponding to two eyes, the nose, and the mouth.

In one embodiment of the present invention, the functional biocellulose mask includes a plurality of pores filled uniformly with the polyphenol-based functional ingredient of the functional solution.

Due to the technical features of the present invention, the functional biocellulose membrane is capable of carrying uniformly a large amount of functional ingredients. Moreover, a facial mask manufactured from the functional membrane of the present invention can greatly facilitate the release of the functional ingredients upon use (at about 35∼37°C) and achieve outstanding skin care effects.

The present invention is further explained in the following drawings and examples. It is understood that the examples given below do not limit the scope of the invention, and it will be evident to those skilled in the art that modifications can be made without departing from the scope of the appended claims.

### BRIEF DESCRIPTION OF THE DRAWINGS

The present invention will be apparent to those skilled in the art from the following detailed description of the preferred embodiments, with reference to the attached drawings, in which:
FIG. 1 is a pictorial diagram showing the functional biocellulose mask of the present invention;
FIGs. 2A-2D are scanning electron micrographs at 10,000x magnification of biocellulose membranes at temperatures of 20°C, 27°C, 37°C, and 60°C, respectively;
FIGs. 2E-2H are scanning electron micrographs at 100,000x magnification of biocellulose membranes at temperatures of 20°C, 27°C, 37°C, and 60°C, respectively;
FIG. 3 shows the results of a diffusion experiment for the functional biocellulose membrane of the present invention at different temperatures; and
FIG. 4 shows the diffusion efficiency of polyphenols released from the functional biocellulose mask of the present invention at 37°C or 25°C.

### DETAILED DESCRIPTION OF THE PREFERRED EMBODIMENT

The present invention provides a method of preparing a functional biocellulose membrane, including immersing a biocellulose membrane in a functional solution, and elevating a temperature of the functional solution to 35°C or higher for a period of time.

The functional solution includes at least a polyphenol-based functional ingredient. The polyphenol-based functional ingredient includes, but not limited to, tea polyphenols such as catechin, anthocyanidins, or isoflavones.

The functional solution may further include at least a pigment. The pigment includes, but not limited to, lutein, carotene, carotenoid, or lycopene. Therefore, the prepared mask products would show different colors and provide customers with different visual experiences. In the case that the functional ingredients in the functional solution are colored, for example, use of tea polyphenols or lycopene, the prepared functional biocellulose membrane offer both the functional effects and the visual effects of the functional ingredients.

The present invention also provides a functional biocellulose mask, whose fibrous structure is filled uniformly with the functional ingredients. The functional biocellulose mask is made by cutting the functional biocellulose membrane, prepared according to the preparation method previously described, into a facial shape. The facial shape indicates a shape with the external contour of the human face and also with openings for the eyes, the nose, and the mouth. When the mask is used, the design of the openings prevents covering these parts of the face with the mask and thus allows the mask to tightly stick to the face. The openings also avoid temporary vision blockage of the users by the mask.

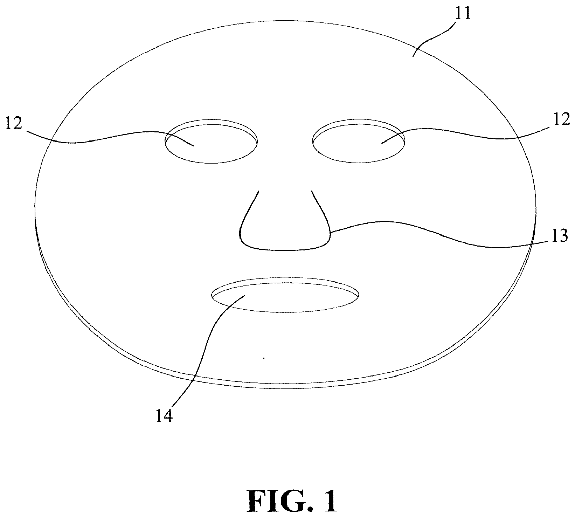

FIG. 1 shows the functional biocellulose mask of the present invention before use. The functional biocellulose mask has a main body **11,** including two openings for eyes **12,** one opening for nose **13,** and one opening for mouth **14** corresponding to two eyes, the nose, and the mouth, respectively. When used, the mask is applied to a user's face to allow its tight adherence to the facial skin, and it is removed from the face after a period of time. This seamless contact at body temperature causes the functional ingredients in the mask to effectively enter the skin, resulting in outstanding skin care effects.

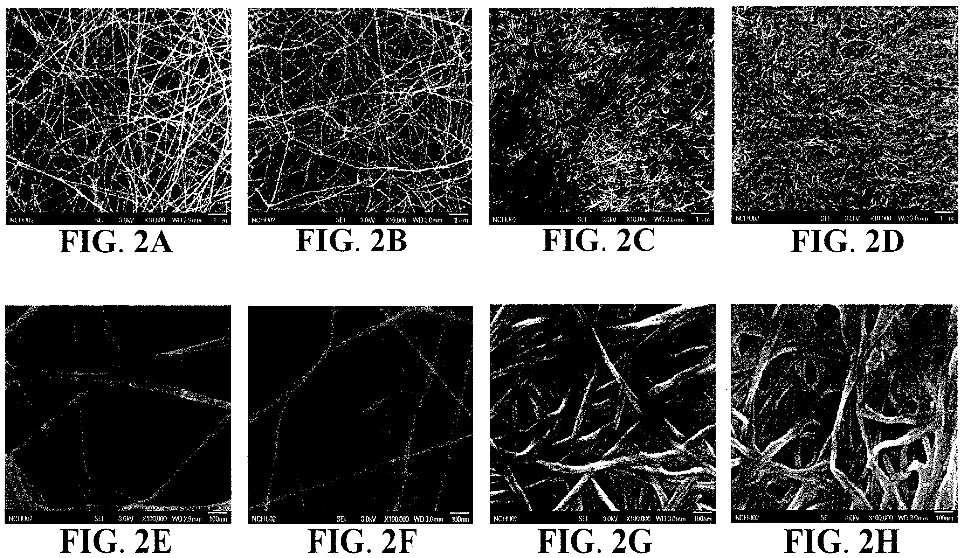

FIGs. 2A-2D and FIGs. 2E-2H are scanning electron micrographs at 10,000x and 100,000x magnifications of biocellulose membranes at temperatures of 20°C, 27°C, 37°C, and 60°C. According to these figures, the fibers of the biocellulose membranes at 37°C and 60°C are thicker than those at 20°C and 27°C, and the fibers at 60°C are even thicker than those at 37°C. The results indicate that the space between the fibers is enlarged at about 37°C or higher. This characteristic of the biocellulose membranes can be utilized in carrying the functional ingredients.

The detailed preparation method of the functional biocellulose membrane is described as follows: drying a biocellulose membrane to have water content of lower than 50 % , placing the dried biocellulose membrane into a functional solution containing at least a functional ingredient, immersing the biocellulose membrane in the functional solution and elevating the temperature of the functional solution to 35°C or higher for a period of time, decreasing the temperature to room temperature after the period of time, and washing out the extra functional solution from the biocellulose membrane with water.

The temperature at which the biocellulose membrane immersed in the functional solution is preferably 35°C∼37°C. This temperature opens up the fibrous structure and facilitates incorporation of the functional ingredients into the biocellulose membrane. The period of time for immersion is preferably 30∼180 minutes. Because the functional ingredients are already locked in the functional biocellulose membrane after the immersion step and the temperature drop, wash of the functional biocellulose membrane for multiple times with water does not wash off the functional ingredients. When the functional biocellulose membrane or the functional biocellulose mask is applied to a user's face, the raised temperature at 35°C∼37°C allows the functional ingredients to be released again, resulting in the skin care effects.

In one embodiment of the present invention, the functional solution contains 0.5∼3% of a green tea extract. The green tea extract includes multiple types of polyphenol-based compounds and provides the biocellulose membrane with antioxidants in the category of polyphenols. In addition to the antioxidant activity, the green tea extract also serves as a source of pigments and offers the refreshing green color.

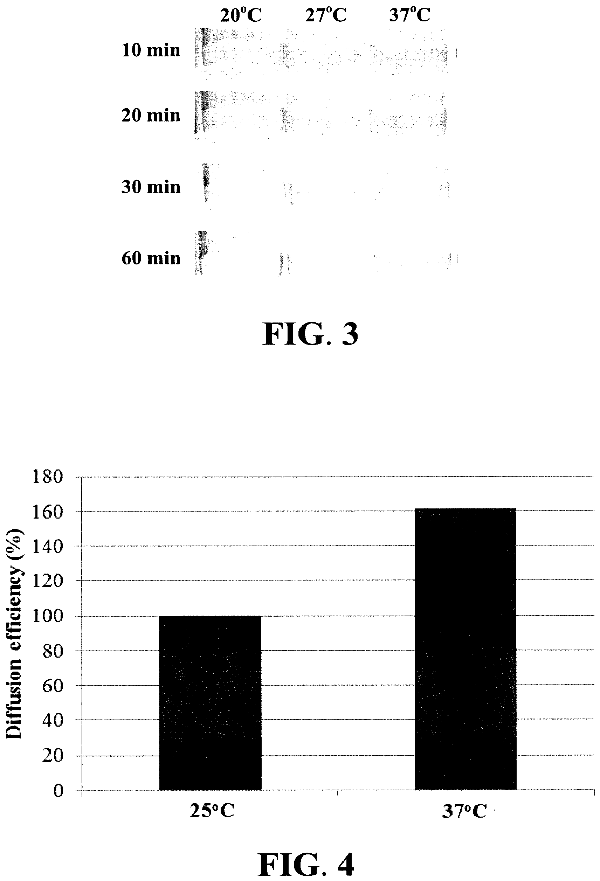

A diffusion experiment was carried out for a predetermined weight of the prepared functional biocellulose membrane (3-5 g) placed in a beaker containing 80∼150 ml distilled water. The temperatures for diffusion were 20°C, 27°C, and 37°C, and the results were examined after 10 minutes, 30 minutes, and 60 minutes of diffusion. The results are shown in FIG. 3, in which a significant increase in the concentration of the green tea extract in the water and a darker color were observed immediately after 10 minutes at 37°C.

Next, in order to investigate the difference in the release of polyphenols from the functional biocellulose membrane of the present invention when it is used (37°C) or stored at room temperature (25°C), a comparison of the release of polyphenols at 37°C and 25°C was made for the functional biocellulose mask.

A predetermined weight of the functional biocellulose masks filled uniformly with tea polyphenols were placed in test tubes added with 10-fold volume of water. The functional biocellulose masks, as triplicates, were immersed in water for 30 minutes at 37°C and 25°C, and then the liquid of these samples was assayed for the phenolic content. Since the time for the immersion process at the two temperatures was the same, the phenolic content of the samples represented the release efficiencies of the functional ingredients from the functional biocellulose mask upon use and in storage.

For determination of the total phenolic content, the following reagents were prepared: Folin-Ciocalteu's phenol reagent at room temperature, 7.5% aqueous solution of sodium carbonate at 4°C prepared from anhydrous sodium carbonate, and 1000 µg/mL aqueous solution of gallic acid at -20°C.

Also, a standard curve of gallic acid was plotted. The 1000 µg/ml aqueous solution of gallic acid is first prepared by pouring 10 mg accurately weighted gallic acid into a 10 mL container followed by addition of water to a final volume of 10 mL. Gallic acid standard solutions at concentrations of 0, 20, 40, 60, 80, and 100 µg/mL were then prepared as shown in TABLE 1.

**TABLE 1**

| Concentration (µg/mL) | **0** | **20** | **40** | **60** | **80** | **100** |
|---|---|---|---|---|---|---|
| Aqueous solution of gallic acid 1,000 ppm | 0 µL | 10 µl | 10 µl | 10 µl | 10 µl | 15 µl |
| water | 100 µL | 490 µL | 240 µL | 156.7 µL | 115 µL | 135 µL |

Each of the gallic acid standard solutions in the amount of 100 µL was then added into a glass test tube, mixed thoroughly with 500 µL Folin-Ciocalteu's phenol reagent, and allowed to stand for 3 minutes. The aqueous solution of 7.5 % sodium carbonate in the amount of 400 µL was added for a 30-minute reaction. After vortexed to eliminate air bubbles, 200 µL of the reaction mixtures was measured for absorbance at 750 nm, and a standard curve was plotted.

The liquid from the samples with the immersed functional biocellulose mask of the present invention was measured for absorbance according to the abovementioned method, and the phenolic content of the liquid was determined by comparison to the standard curve. The results are shown in FIG. 4. When compared with the phenolic content of the samples treated at 25°C, the phenolic content of the samples with diffused tea polyphenols at 37°C was increased by 61%. In other words, there was a 61% increase in the diffusion efficiency of polyphenols from the functional biocellulose mask at 37°C. The result indicated that significantly more functional ingredients were released when the functional biocellulose mask was used (37°C).

In conclusion, the functional biocellulose membrane of the present invention can effectively lock the functional ingredients in the biocellulose fibers. Therefore, the functional ingredients are able to be uniformly distributed in all pores of the functional biocellulose membrane. Moreover, according to the phenol-release experiment previously described, the functional biocellulose mask, which is prepared from the functional biocellulose membrane of the present invention, can achieve much greater efficiency of releasing functional ingredients when it is used at close to body temperature (35°C∼37°C), leading to outstanding skin care effects.

The present invention has been described with reference to the above preferred embodiments. However, it will be apparent to those skilled in the art that modifications and changes in form and detail may be made without departing from the scope of the present invention defined by the appended claims.

## Claims

1. A method of preparing a functional biocellulose membrane, comprising immersing a biocellulose membrane in a functional solution, and
elevating a temperature of the functional solution to 35°C or higher for a period of time.

2. The method of claim 1, wherein the functional solution comprises at least a polyphenol-based functional ingredient.

3. The method of claim 2, wherein the polyphenol-based functional ingredient is selected from the group consisting of tea polyphenols, anthocyanidins, and isoflavones.

4. The method of claim 3, wherein the tea polyphenol is catechin.

5. The method of claim 1, wherein the functional solution further comprises at least a pigment selected from the group consisting of lutein, carotenes, and carotenoids.

6. The method of claim 5, wherein the carotenoid is lycopene.

7. The method of claim 1, wherein the temperature is 35°C∼37°C.

8. The method of claim 1, wherein the period of time is 30-180 minutes.

9. A functional biocellulose mask, which is a biocellulose membrane prepared according to the method of claim 1 and has a facial shape, comprising a plurality of openings corresponding to two eyes, the nose, and the mouth.

10. The functional biocellulose mask of claim 9, wherein the functional solution comprises at least a polyphenol-based functional ingredient.

11. The functional biocellulose mask of claim 10, wherein the polyphenol-based functional ingredient is selected from the group consisting of tea polyphenols, anthocyanidins, and isoflavones.

12. The functional biocellulose mask of claim 11, wherein the tea polyphenol is catechin.

13. The functional biocellulose mask of claim 9, wherein the functional solution further comprises at least a pigment selected from the group consisting of lutein, carotenes, and carotenoids.

14. The functional biocellulose mask of claim 13, wherein the carotenoid is lycopene.

15. The functional biocellulose mask of claim 10, which comprises a plurality of pores filled uniformly with the polyphenol-based functional ingredient of the functional solution.

16. The functional biocellulose mask of claim 15, wherein the functional biocellulose mask releases the polyphenol-based functional ingredient at a temperature above 35°C.
